(19) Europäisches Patentamt
European Patent Office
Office européen des brevets

(11) Numéro de publication: **0 424 214 A1**

(12) **DEMANDE DE BREVET EUROPEEN**

(21) Numéro de dépôt: **90402820.6**

(22) Date de dépôt: **10.10.90**

(51) Int. Cl.5: **C07D 281/10**, C07D 417/12, A61K 31/55

(30) Priorité: **17.10.89 FR 8913540**

(43) Date de publication de la demande: **24.04.91 Bulletin 91/17**

(84) Etats contractants désignés: **AT BE CH DE DK ES FR GB GR IT LI LU NL SE**

(71) Demandeur: **SYNTHELABO**
**58 rue de la Glacière**
**F-75013 Paris(FR)**

(72) Inventeur: **Muller, Jean-Claude**
**4, Avenue de l'Espérance**
**F-91390 Morsang Sur Orge(FR)**
Inventeur: **Lassalle, Gilbert**
**157Bis, avenue Jean-Jaurès**
**F-92140 Clamart(FR)**
Inventeur: **Denys, Colombe**
**7, rue André Theuriet**
**F-92340 Bourg la Reine(FR)**
Inventeur: **Lochead, Alistair**
**19, rue Arthur Croquette**
**F-94220 Charenton-le-Pont(FR)**

(74) Mandataire: **Ludwig, Jacques et al**
**SYNTHELABO, Service Brevets, 22, avenue Galilée, B.P. 72**
**F-92352 Le Plessis Robinson Cédex(FR)**

(54) **Dérivés de 5-[2-[[2-amino-2-oxoéthyl]méthylamino]éthyl]-2,3-dihydro-3-hydroxy-2-(4-méthoxyphényl)-1,5(5H)-benzothiazépine-4-one, leur préparation et leur application en thérapeutique.**

(57) Composés, sous forme de diastéréiosomères purs ou de leurs mélanges, répondant à la formule générale (I)

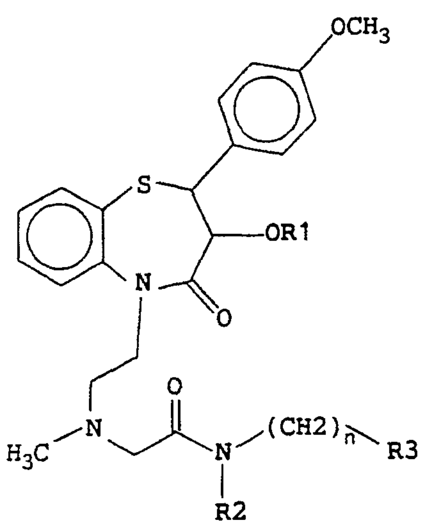

(I)

dans laquelle R1 représente un atome d'hydrogène ou un groupe alcanoyle en $C_2$-$C_4$, R2 représente un atome d'hydrogène ou un groupe alkyle en $C_1$-$C_4$, n représente un nombre de 0 à 3, et R3 représente soit un groupe phényle portant de 1 à 3 substituants choisis parmi les atomes d'halogène et les groupes trifluorométhyle,

EP 0 424 214 A1

méthyle, alcoxy ou alkylthio en $C_1$-$C_4$, nitro, cyano, carbamoyle, acétamido, méthanesulfonamido, méthoxycarbonyle ou phénylcarbonyle, soit un groupe hétérocyclique aromatique tel qu'un groupe pyridinyle ou imidazolyle.
Application en thérapeutique.

## DERIVES DE 5-[2-[[2-AMINO-2-OXOETHYL]METHYLAMINO]ETHYL]-2,3-DIHYDRO-3-HYDROXY-2-(4-METHOXYPHENYL)-1,-5(5H )-BENZOTHIAZEPINE-4-ONE, LEUR PREPARATION ET LEUR APPLICATION EN THERAPEUTIQUE

La présente invention a pour objet des dérivés de 5-[2-[[2-amino-2-oxoéthyl]méthylamino]éthyl]-2,3-dihydro-3-hydroxy-2-(4-méthoxyphényl)-1,5(5H )-benzothiazépine-4-one, leur préparation et leur application en thérapeutique.

Les composés de l'invention répondent à la formule générale (I) donnée dans le schéma ci-après, formule dans laquelle R1 représente un atome d'hydrogène ou un groupe alcanoyle en $C_2$-$C_4$,
R2 représente un atome d'hydrogène ou un groupe alkyle en $C_1$-$C_4$,
n représente un nombre entier de 0 à 3, et
R3 représente soit un groupe phényle portant de 1 à 3 substituants choisis parmi les atomes d'halogène et les groupes trifluorométhyle, méthyle, alcoxy ou alkylthio en $C_1$-$C_4$, nitro, cyano, carbamoyle, acétamido, méthanesulfonamido, méthoxycarbonyle ou phénylcarbonyle, soit un groupe hétérocyclique aromatique tel qu'un groupe pyridinyle ou imidazolyle.

Les composés de formule générale (I) peuvent se présenter à l'état de bases libres ou de sels d'addition à des acides. Les atomes de carbone aux positions 2 et 3 étant asymétriques, ils peuvent aussi exister sous diverses formes diastéréoisomères, racémiques ou optiquement pures. Ces diverses formes font partie de l'invention.

Conformément à l'invention on peut préparer les composés de formule générale (I) selon le schéma donné ci-après.

Une première méthode consiste à faire réagir une benzothiazépinone de formule (V) (dans laquelle R1 est tel que défini ci-dessus) avec une amine halogénée de formule générale (IV) (dans laquelle R2 et R3 sont tels que définis ci-dessus et Y est un atome d'halogène tel que le chlore ou le brome). La seconde méthode consiste à faire réagir une benzothiazépinone de formule (VI) (dans laquelle R1 est tel que défini ci-dessus) avec un dérivé halogéné de formule générale (II) (dans laquelle R2 et R3 sont tels que définis ci-dessus et Y est un atome d'halogène tel que le chlore ou le brome).

SCHEMA

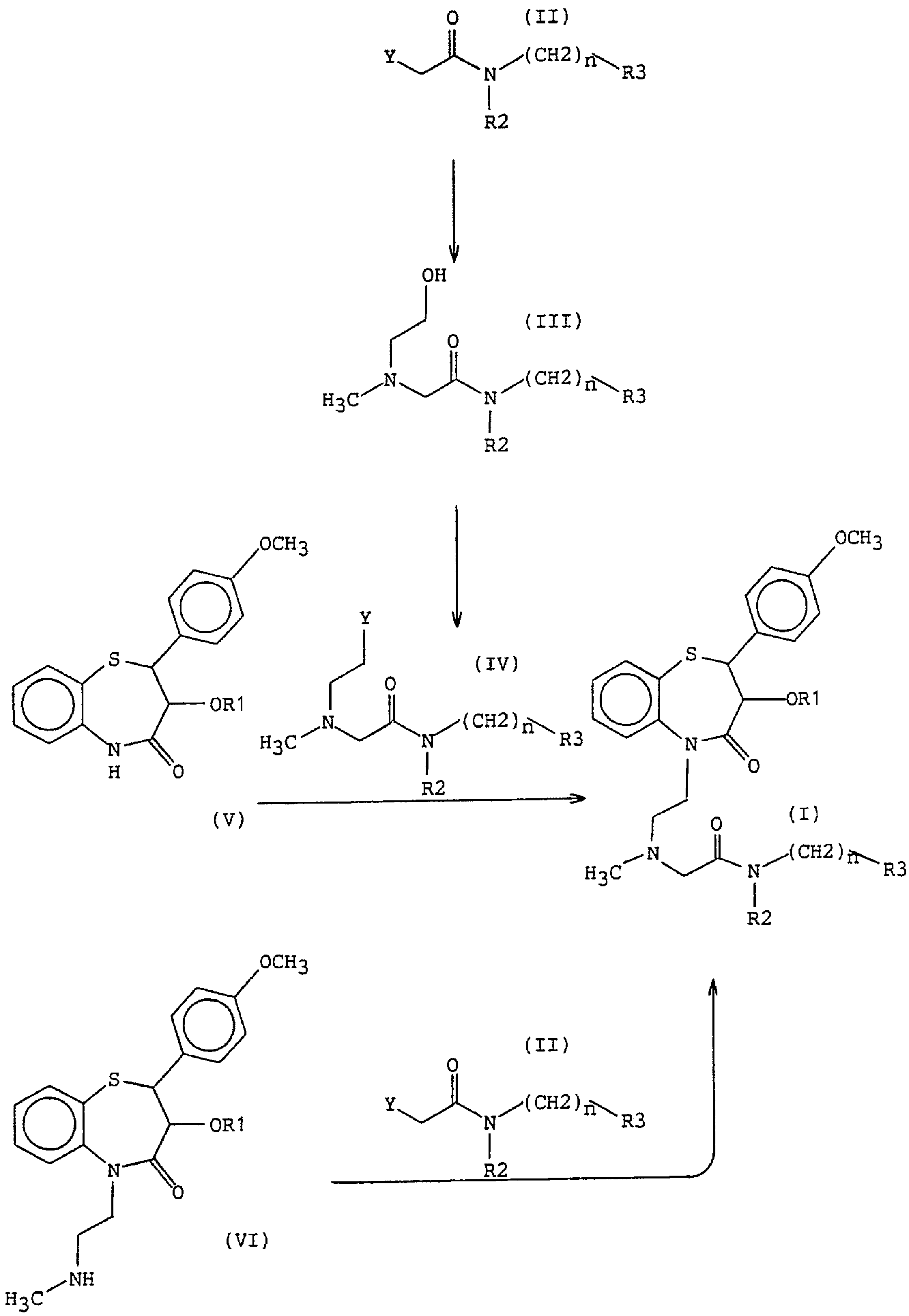

(II)

(III)

(IV)

(V)

(I)

(II)

(VI)

Ces deux réactions, entre soit une benzothiazépinone (V) soit une amine (VI) (sous forme de base libre ou de sel d'addition) et un dérivé halogéné, sont classiques, et se déroulent donc dans des conditions bien connues de l'homme du métier, c,est-à-dire par exemple dans un solvant aprotique, tel que l'acétone, la butanone-2 ou l'acétate d,éthyle, à la température du reflux, en présence d'une base destinée à fixer l'acide libéré (et, le cas échéant, à libérer la base du composé de départ), par exemple le carbonate de potassium, et éventuellement en présence d'un catalyseur de transfert de phase tel que l'iodure de tétra-n-butylammonium ou le chlorure de benzyltriéthylammonium.

Il va de soi que l'on peut transformer un composé de formule (I) dans laquelle R1 représente un atome d'hydrogène en un composé de formule (I) dans laquelle R1 représente un groupe alcanoyle par une acylation de type connu, et que la transformation inverse est possible par hydrolyse.

Les benzothiazépinones de formule (V) sont décrites dans le brevet des Etats-Unis d'Amérique N° 3562257 ; celles de formule (VI) sont décrites dans les demandes de brevets européens N° 158339 et 158340.

L'amine halogénée de formule (IV) peut être préparée par exemple à partir de l'alcool de formule (III), par action d'un agent d'halogénation tel que le chlorure de thionyle. L'aminoalcool de formule (III) peut lui même être préparé selon toute méthode connue, par exemple à partir d'un dérivé halogéné de formule (II) et de méthylamino-2 éthanol. Les composés de formule (II) sont accessibles à partir de chlorure de chloroacétyle et d'amines de formule générale $R2\text{-}HN\text{-}(CH_2)_n\text{-}R3$ (dans laquelle R2, n et R3 sont tels que définis ci-dessus), qui sont décrites dans la littérature.

Les exemples qui vont suivre illustrent en détail la préparation de quelques composés conformes à l'invention. Les microanalyses élémentaires et les spectres IR et RMN confirment les structures des produits obtenus. Les numéros indiqués entre parenthèses pour chaque exemple correspondent à ceux du tableau qui suit, et qui illustre les structures et propriétés physiques de quelques composés selon l'invention.

Exemple 1 (Composé n° 1)

(+)-cis-(2S,3S)-3-Acétyloxy-2,3-dihydro-5-[2-[[2-(3,4-diméthoxyphényl)amino-2-oxoéthyl]méthylamino]éthyl]-2-(4-méthoxyphényl)-1,5(5*H*)-benzothiazépine-4-one.

On chauffe au reflux pendant 9h un mélange de 4 g (9,15 mmoles) de chlorhydrate de (+)-cis-(2S,3S)-3-acétyloxy-2,3-dihydro-2-(4-méthoxyphényl)-5-(2-methylaminoéthyl)-1,5(5*H*)-benzothiazépine-4-one, 2,4 g (10 mmoles) de 1-chloro-*N*-(3,4-diméthoxyphényl)acétamide et 9,9 g de carbonate de potassium dans 50 ml de butanone-2.

On filtre le mélange, on évapore le filtrat, et on purifie le résidu par chromatographie sur colonne de silice. On obtient ainsi 5,3 g d'huile qu'on reprend avec 60 ml d'éthanol et qu'on traite avec 0,81 g d'acide oxalique. On filtre le précipité obtenu par addition d'éther, et on le recristallise dans l'éthanol. On isole 4 g d'oxalate.

Point de fusion : 144° C $[\alpha]_D^{20} = +59,4°$ (c=0,5 ; MeOH).

Exemple 2 (Composé n° 11)

(+)-cis-(2S,3S)-3-Acétyloxy-2,3-dihydro-5-[2-[[2-[(4-méthylphényl)méthyl]méthylamino-2-oxoéthyl]-méthylamino]éthyl]-2-(4-méthoxyphényl)-1,5(5*H*)-benzothiazépine-4-one.

On chauffe au reflux pendant 9h un mélange de 4 g (9,15 mmoles) de chlorhydrate de (+)-cis-(2S,3S)-3-acétyloxy-2,3-dihydro-2-(4-méthoxyphényl)-5-(2-méthylaminoéthyl)-1,5(5H)-benzothiazépine-4-one, 1,85 g (8,73 mmoles) de 1-chloro-*N*-méthyl-*N*-[(4-méthylphényl)méthyl]acétamide et 6,05 g de carbonate de potassium dans 50 ml de butanone-2. On filtre le mélange, on évapore le filtrat et on purifie le résidu par chromatographie sur colonne de silice. On obtient ainsi 3,5 g d'huile qu'on reprend dans l'éthanol et qu'on traite avec 1 équivalent d'acide oxalique. On filtre le précipité obtenu par addition d'éther, et on le recristallise dans l'éthanol. On isole 2,5 g d'oxalate.

Point de fusion : 108,3-109,2° C $[\alpha]_D^{20} = +71,4°$ (c=0,3 ; MeOH).

Exemple 3 (Composé n° 18).

( + )-cis-(2S,3S)-3-Acétyloxy-2,3-dihydro-5-[2-[[2-[2-(3,4-diméthoxyphényl)éthyl]méthylamino-2-oxoéthyl]-méthylamino]éthyl]-2-(4-méthoxyphényl)-1,5(5H )-benzothiazépine-4-one.

On chauffe au reflux pendant 8h un mélange de 3 g (6,7 mmoles) de chlorhydrate de ( + )-cis-(2S,3S)-3-acétyloxy-2,3-dihydro-2-(4-méthoxyphényl)-5-(2-méthylaminoéthyl)-1,5(5H )-benzothiazépine-4-one, 2,4 g (8,9 mmoles) de 1-chloro-N -méthyl-N -[2-(3,4-diméthoxyphényl)éthyl]acétamide et 7 g de carbonate de potassium dans 50 ml de butanone-2. On filtre le mélange, on évapore le filtrat et on purifie le résidu par chromatographie sur colonne de silice. On obtient une huile qu'on reprend dans l'éthanol et qu'on traite avec 1 équivalent d'acide oxalique. On filtre le précipité obtenu par addition d'éther, et on le recristallise dans l'éthanol. On isole 2 g d'oxalate.
Point de fusion : 90° C $[\alpha]_D^{20}$ = .52,8° (c = 0,5 ; MeOH).

Exemple 4 (Composé n° 20).

( + )-cis-(2S,3S)-3-Acétyloxy-2,3-dihydro-5-[2-[[2-[3-(1-imidazolyl)propyl]amino-2-oxoéthyl]méthylamino]-éthyl]-2-(4-méthoxyphényl)-1,5(5H )-benzothiazépine-4-one.

On chauffe au reflux pendant 10h un mélange de 4 g (9,15 mmoles) de chlorhydrate de ( + )-cis-(2S,3S)-3-acétyloxy-2,3-dihydro-2-(4-méthoxyphényl)-5-(2-méthylaminoéthyl)-1,5(5H )-benzothiazépine-4-one, 4 g (19,8 mmoles) de 1-chloro-N -[3-(1-imidazolyl)propyl]acétamide et 3,6 g de carbonate de potassium dans 60 ml de butanone-2.
On filtre le mélange, on évapore le filtrat, et on purifie le résidu par chromatographie sur colonne de silice. On obtient ainsi 2 g d'huile qu'on reprend avec 12 ml d'éthanol et qu'on traite avec 0,32 g d'acide oxalique. On filtre le précipité obtenu par addition d'éther, et on le recristallise dans l'éthanol. On isole 1,1 g d'oxalate.
Point de fusion : 55° C $[\alpha]_D^{20}$ = + 51,3° (c = 0,5 ; MeOH).

Tableau

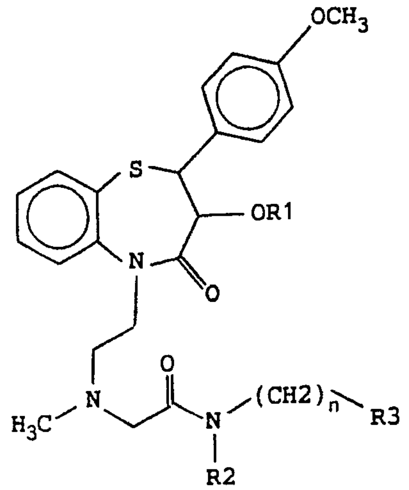

(I)

| N° | R1 | R2 | n | R3 | Sel | $[\alpha]_D^{20}$ c(%), MeOH | F(°C) |
|---|---|---|---|---|---|---|---|
| 1 | $COCH_3$ | H | 0 | $C_6H_3$-3,4-$(OCH_3)_2$ | ox. | +59,4 (0,5) | 144 |
| 2 | $COCH_3$ | H | 0 | $C_6H_4$-2-$COC_6H_5$ | ox. | +90,1 (0,5) | 85 |
| 3 | $COCH_3$ | $CH_3$ | 0 | $C_6H_3$-3,4-$(OCH_3)_2$ | ox. | +54,0 (0,1) | 120 |
| 4 | $COCH_3$ | H | 1 | $C_6H_3$-3,4-$(OCH_3)_2$ | ox. | +84,1 (0,5) | 190 |
| 5 | $COCH_3$ | $CH_3$ | 1 | $C_6H_4$-3-$OCH_3$ | ox. | +75,3 (0,3) | 106 |
| 6 | $COCH_3$ | $CH_3$ | 1 | $C_6H_3$-3-Cl | ox. | +73,0 (0,5) | 95 |
| 7 | $COCH_3$ | $CH_3$ | 1 | $C_6H_3$-3,4-$(OCH_3)_2$ | ox. | +69,4 (0,5) | 115 |

Tableau (suite)

| N° | R1 | R2 | n | R3 | Sel | $[\alpha]_D^{20}$ c(%), MeOH | F(°C) |
|---|---|---|---|---|---|---|---|
| 8 | $COCH_3$ | $CH_3$ | 1 | $C_6H_3$-3,4-$Cl_2$ | ox. | +71,6 (0,5) | 125 |
| 9 | $COCH_3$ | $CH_3$ | 1 | $C_6H_3$-3,4-$(CH_3)_2$ | ox. | +69,8 (0,5) | 92-95,5 |
| 10 | $COCH_3$ | $CH_3$ | 1 | $C_6H_2$-3,4,5-$(OCH_3)_3$ | ox. | +67,6 (0,4) | 143,4-144,6 |
| 11 | $COCH_3$ | $CH_3$ | 1 | $C_6H_4$-4-$CH_3$ | ox. | +71,4 (0,3) | 108,3-109,2 |
| 12 | $COCH_3$ | $CH_3$ | 1 | $C_6H_4$-4-$CF_3$ | ox. | +69,0 (0,2) | 126,2-128,3 |
| 13 | $COCH_3$ | $CH_3$ | 1 | $C_6H_4$-4-F | ox. | +72,8 (0,1) | 86-88 |
| 14 | $COCH_3$ | $CH_3$ | 1 | $C_6H_4$-4-On$C_4H_9$ | ox. | +64,9 (0,2) | 87,5-91,5 |
| 15 | $COCH_3$ | $CH_3$ | 1 | $C_6H_4$-4-$SCH_3$ | ox. | +65,0 (0,2) | 128,5-129,5 |
| 16 | $COCH_3$ | $CH_3$ | 2 | $C_6H_4$-3-$OCH_3$ | ox. | +80,4 (0,5) | 85 |
| 17 | $COCH_3$ | $CH_3$ | 2 | 2-pyridinyle | ox. | +70,9 (0,5) | 85 |
| 18 | $COCH_3$ | $CH_3$ | 2 | $C_6H_3$-3,4-$(OCH_3)_2$ | ox. | +52,9 (0,5) | 90 |

Tableau (fin)

| N° | R1 | R2 | n | R3 | Sel | $[\alpha]_D^{20}$ c(%),MeOH | F(°C) |
|---|---|---|---|---|---|---|---|
| 19 | $COCH_3$ | $CH_3$ | 2 | $C_6H_2$-3,4,5-$(OCH_3)_3$ | ox. | +65,4 (0,5) | 100 |
| 20 | $COCH_3$ | H | 3 | 1-imidazolyle | ox. | +51,3 (0,5) | 55 |
| 21 | $COCH_3$ | $CH_3$ | 1 | $C_6H_4$-4-$OCH_3$ | ox. | +68,4 (0,2) | 90,5 |
| 22 | $COCH_3$ | $CH(CH_3)_2$ | 1 | $C_6H_4$-4-$CH_3$ | fum | +91,8 (0,2) | 121-123 |
| 23 | $COCH_3$ | $CH_3$ | 1 | $C_6H_4$-4-$CO_2CH_3$ | fum | +72,5 (0,2) | 129-131 |
| 24 | $COCH_3$ | $CH_3$ | 1 | $C_6H_4$-4-Cl | fum | +85,0 (0,2) | 129-130 |
| 25 | $COCH_3$ | $CH_3$ | 1 | $C_6H_4$-3-Br-4-$CH_3$ | ox. | +61,0 (0,2) | 119-122 |
| 26 | $COCH_3$ | $CH_3$ | 1 | $C_6H_4$-4-CN | fum | +75,5 (0,2) | 96 |
| 27 | $COCH_3$ | $CH_3$ | 1 | $C_6H_4$-4-$NO_2$ | fum | +76,2 (0,2) | 110-112 |

Légende : dans la colonne "sel", ox. désigne un oxalate et fum désigne un fumarate.

Les composés de l'invention ont fait l'objet d'une série d'essais pharmacologiques qui ont mis en évidence leur intérêt comme substances thérapeutiques du système cardiovasculaire.

Leur activité comme antagonistes du calcium a été montrée grâce à un essai sur l'aorte isolée de lapin. Le protocole expérimental utilisé est une variante de celui utilisé par Godfraind et Kaba (1969), (Blockade or reversal of the contraction induced by calcium and adrenaline in depolarized arterial smooth muscle, Br. J. Pharmac., 36 , 549-560). Le détail de l'essai est décrit dans le brevet européen N° 0103500.
On calcule la concentration molaire provoquant 50% de décontraction de la réponse au calcium ($CE_{50}$), ou bien son antilogarithme ($pCE_{50}$).
Les $pCE_{50}$ des composés de l'invention vont de 4,5 à 6,6.

Les composés de l'invention ont aussi fait l'objet d'un essai de liaison (binding) de $[^3H]$nitrendipine sur le cortex total de rat.
Le détail de l'essai est décrit dans la demande de brevet européen N° 0300865.
Les concentrations $CI_{50}$ des composés de l'invention (concentrations qui inhibent 50% de la liaison spécifique de la $^3[H]$nitrendipine) se situent entre 0,001 et 10 $\mu$M.

Les composés de l'invention ont encore fait l'objet d'un essai d'inhibition de la liaison spécifique du "PAF", facteur d'activation des plaquettes du sang, essai dont le protocole est décrit dans la demande de brevet européen N° 0320362.
Les $CI_{50}$ (concentrations qui inhibent de 50% la liaison spécifique) des composés de l'invention, dans cet essai, se situent entre 0,5 et 5 $\mu$M.

Enfin les composés de l'invention ont fait l'objet d'un essai d'inhibition de l'agrégation des plaquettes du sang induite par le "PAF-acéther" (1-O-($C_{16}$-$C_{18}$-alkyl)-2-acétyl- sn-glycéryl-3-phosphorylcholine), essai dont le protocole est également décrit dans la demande de brevet européen N° 0320362.
L'action anti-agrégante des composés s'exprime par la concentration $IC_{50}$, concentration qui inhibe de 50% l'agrégation provoquée par le PAF-acéther.
Les $CI_{50}$ des composés de l'invention, dans cet essai, se situent entre entre 2 et 20 $\mu$M.

Les résultats des essais montrent que les composés de l'invention sont des antagonistes du calcium et peuvent, à ce titre, être utilisés pour le traitement de diverses affections pour lesquelles ce type d'agents est indiqué. C'est ainsi qu'en particulier ils peuvent être utilisés en médecine cardiovasculaire pour le traitement d'affections nécessitant des modulateurs des mouvements transmembranaires et intracellulaires du calcium, tout spécialement l'hypertension, l'angor et l'arythmie cardiaque.
Ils sont, en outre, susceptibles de présenter des effets antiathérogènes, anti-ischémiques cardiaques, anti-ischémiques cérébraux, antimigraineux, antiépileptiques, anti-asthmatiques et antiulcéreux.
Dans le domaine cardiovasculaire ils peuvent être utilisés seuls ou associés à d'autres substances actives connues telles que les diurétiques, les $\beta$-bloquants, les inhibiteurs de l'enzyme de conversion de l'angiotensine, les antagonistes des récepteurs $\alpha_1$.
Ils peuvent être également indiqués pour potentialiser l'effet ou diminuer la toxicité d'agents utilisés dans le traitement du cancer ou dans les transplantations d'organes.

Les composés de l'invention peuvent être présentés sous toutes formes appropriées à l'administration orale ou parentérale, en association avec des excipients connus, par exemple sous forme de comprimés, gélules, dragées, capsules, solutions ou suspensions buvables ou injectables.
La posologie journalière peut aller par exemple de 30 à 300 mg par la voie orale et de 25 à 100 mg par voie parentérale.

**Revendications**

1. Composés, sous forme de diastéréoisomères purs ou de leurs mélanges, répondant à la formule générale (I)

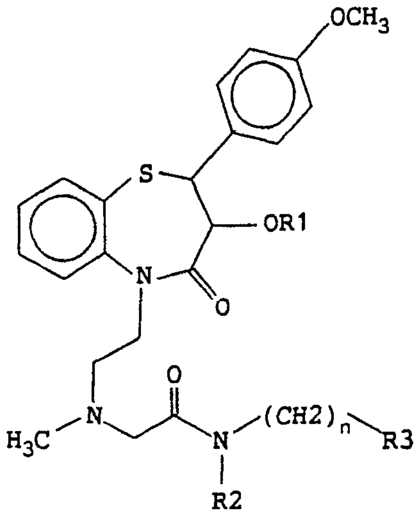

(I)

dans laquelle
R1 représente un atome d'hydrogène ou un groupe alcanoyle en $C_2$-$C_4$,
R2 représente un atome d'hydrogène ou un groupe alkyle en $C_1$-$C_4$,
n représente un nombre entier de 0 à 3, et
R3 représente soit un groupe phényle portant de 1 à 3 substituants choisis parmi les atomes d'halogène et les groupes trifluorométhyle, méthyle, alcoxy ou alkylthio en $C_1$-$C_4$, nitro, cyano, carbamoyle, acétamido, méthanesulfonamido, méthoxycarbonyle ou phénylcarbonyle, soit un groupe hétérocyclique aromatique tel qu'un groupe pyridinyle ou imidazolyle,
ainsi que leurs sels d'addition à des acides acceptables en pharmacologie.

2. Procédé de préparation des composés selon la revendication 1, caractérisé en ce qu'on fait réagir une benzothiazépinone de formule générale (VI)

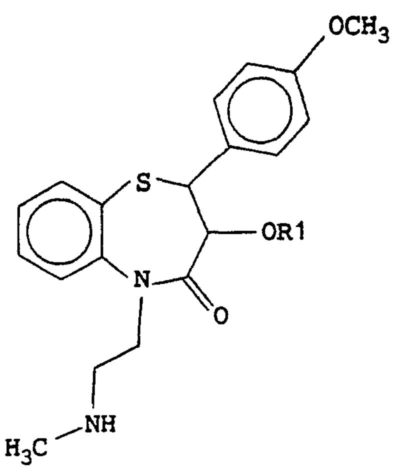

(VI)

(dans laquelle R1 est tel que défini dans la revendication 1)
avec un composé de formule générale (II)

(II)

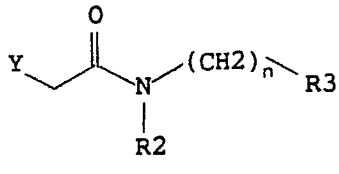

(dans laquelle R2 et R3 sont tels que définis dans la revendication 1 et Y représente un atome d'halogène).
3. Composition pharmaceutique, caractérisée en ce qu'elle contient un composé selon l'une des revendications 1 et 2, associé à un excipient pharmaceutique.

Revendications pour les Etats contractants suivants: ES,GR

Procédé de préparation de composés, sous forme de diastéréoisomères purs ou de leurs mélanges, répondant à la formule générale (I)

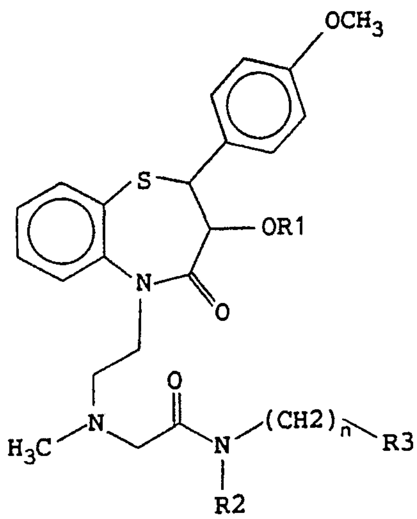

(I)

dans laquelle
R1 représente un atome d'hydrogène ou un groupe alcanoyle en $C_2$-$C_4$,
R2 représente un atome d'hydrogène ou un groupe alkyle en $C_1$-$C_4$,
n représente un nombre entier de 0 à 3, et
R3 représente soit un groupe phényle portant de 1 à 3 substituants choisis parmi les atomes d'halogène et les groupes trifluorométhyle, méthyle, alcoxy ou alkylthio en $C_1$-$C_4$, nitro, cyano, carbamoyle, acétamido, méthanesulfonamido, méthoxycarbonyle ou phénylcarbonyle, soit un groupe hétérocyclique aromatique tel qu'un groupe pyridinyle ou imidazolyle,
procédé caractérisé en ce qu'on fait réagir une benzothiazépinone de formule générale (VI)

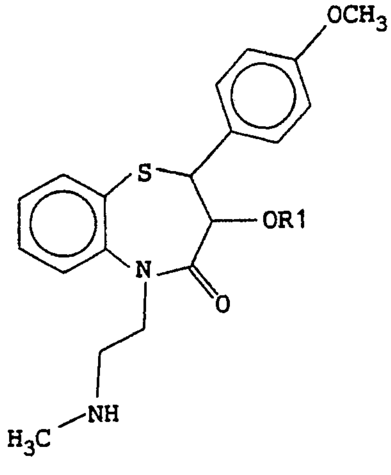

(VI)

(dans laquelle R1 est tel que défini ci-dessus) avec un composé de formule générale (II)

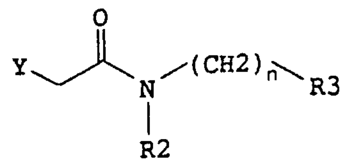

(II)

(dans laquelle R2 et R3 sont tels que définis ci-dessus et Y représente un atome d'halogène).

Office européen des brevets

# RAPPORT DE RECHERCHE EUROPEENNE

Numéro de la demande

**EP 90 40 2820**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 320 362 (SYNTHELABO)<br>* En entier *<br>- - - | 1-3 | C 07 D 281/10<br>C 07 D 417/12<br>A 61 K 31/55 |
| Y | FR-A-2 110 275 (HOFFMANN-LA ROCHE)<br>* En entier *<br>- - - | 1-3 | |
| P,X | EP-A-0 338 892 (SYNTHELABO)<br>* En entier, et en particulier page 8, no. 5,7,8; page 9, no. 9 *<br>- - - - - | 1-3 | |

DOMAINES TECHNIQUES RECHERCHES (Int. Cl.5)

C 07 D 281/00
C 07 D 417/00

Le présent rapport de recherche a été établi pour toutes les revendications

| Lieu de la recherche | Date d'achèvement de la recherche | Examinateur |
|---|---|---|
| La Haye | 23 janvier 91 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

&: membre de la même famille, document correspondant